# EUROPEAN PATENT SPECIFICATION

(11) **EP 4 291 332 B1**
(45) Date of publication and mention of the grant of the patent: **18.12.2024**
(21) Application number: 22706914.3
(22) Date of filing: 09.02.2022
(51) Int. Cl.: B01L 3/00, A61B 10/00, B65B 3/00, B65B 31/04, B65B 59/00, B65B 55/02, B65B 55/22, G01N 1/31, B65B 7/28

(54) **CONTAINER LID PROVIDED WITH A CLOSABLE THROUGH OPENING**
BEHÄLTERDECKEL MIT VERSCHLIESSBARER DURCHGANGSÖFFNUNG
COUVERCLE DE RÉCIPIENT POURVU D'UNE OUVERTURE TRAVERSANTE POUVANT ÊTRE FERMÉE

(30) Priority: 10.02.2021 IT 202100002909
(43) Date of publication of application: 20.12.2023
(73) Proprietor: Bios - Health Diagnostics S.r.l., 57123 Livorno (IT)
(72) Inventor: BICOCCHI, Enrico, 57125 Livorno (IT)
(74) Representative: Cardelli, Guido
(86) International application number: PCT/IB2022/051166
(87) International publication number: WO 2022/172171

(56) References cited:
- WO-A1-2018/179015
- WO-A2-2020/016727
- US-A1- 2014 213 934
- US-A1- 2018 128 718

## Description

### Technical Field

The present invention relates to a container lid provided with a closable through opening.

In particular, the lid is suitable for a disposable container, apt to contain a sample of biological tissue immersed in a fixative-preservative substance introduced into it, in conditions of safety for the healthcare worker. The fixative-preservative substance can be a fixative-preservative solution; among them the most used are those which, since they contain substances known to be toxic by contact and inhalation, must by law be used and transported without any risk of exposure for health workers.

### Background Art

US 2018/018718 A1 discloses a container lid provided with a closable through opening, and a closing member that is spring loaded along an axis coincident with the axis of the through opening. The through opening is made in the lid wall.

WO 2020/016727 A1 describes a system for preserving a surgically explanted tissue sample, comprising a first packing station for placing the tissue sample in a container like that one above cited, and a final packing station in which the container is filled with the preservative substance. The filling system includes a nozzle-holder head mounted movable on a vertical guide; the nozzle-holder head has at least one nozzle for filling the selected container. Further, WO 2020/016727 A1 discloses said container with a hermetic lid. The lid is provided with an opening equipped with a non-return valve and the nozzle is suitable to be inserted inside the non-return valve. In the international patent application above cited, the non-return valve comprises a lower part positioned under the lid and containing a spiral spring and a valve cap, the lower part having a hole on the bottom and side openings, and an upper part joined to the lower part and positioned above the lid, being provided with a hole concentric to the hole in the lower part in a recess intended to be closed by the valve cap, after that the nozzle has been withdrawn outside the valve.

The container according to the aforementioned international application is described only schematically, and the present invention aims to solve some manufacturing problems and drawbacks that could occasionally arise in its use.

WO 2018/1790015 A1 discloses a system for collecting biopsy specimens. It comprises an upper container adapted to contain a liquid, a lower container, which can be sealed in a sealed manner with the upper container, and valve means suitable for allowing or interrupting on command the flow of the liquid from the upper container towards and within the lower container.

US 2014/0213934 A1 describes a specimen collection container having a fluid separation chamber for receiving a fluid stream and for separating an initial volume of the fluid stream from a midstream portion of the fluid stream. The container includes a first chamber and a second chamber, a valve being disposed between the first chamber and the second chamber. A method for collecting a fluid sample is also disclosed.

### Summary of Invention

An object of the present invention is to provide a container lid, in particular for in vitro diagnostics, having the function of preserving and transporting a tissue or parts of human or animal organs to be subjected to histopathological examinations of morphological, immunohistochemical and molecular type.

In particular, an object of the invention is to provide a lid for a disposable container with hermetic closure, container that can allow to contain an explanted sample in a first packing step directly inside the surgery room without risk of biological pollution or of fall during the subsequent transport, and to fill the container in a second packing phase with a fixative-preservative substance that cannot be dispersed into the environment, not even as an aeriform.

Yet, another object of the present invention is to provide a lid for hermetically sealed container characterized by simplicity of use, capable of avoiding risks of exposure by both contact and inhalation, when the preservative substance is a fixative-preservative solution, typically containing formaldehyde and/or other toxic or noxious substances.

A practical object of the invention is to provide a lid for hermetically sealed container that can be easy to build and cheap.

Another object of the invention is to provide a container lid having a through opening, which can be reliably opened and closed automatically by a machine nozzle, machine intended to dispense a fixative-preservative substance inside the container into which the sample of tissue or of parts of human or animal organ is already present.

These and other objects are achieved by a container lid equipped with a closable through opening, described in claim 1 and in claims dependent onto.

### Brief Description of Drawings

Further characteristics and advantages of the present invention will become most clear from the description of embodiments of a container lid for a container of a surgically explanted tissue sample as illustrated by way of example in the accompanying drawings in which:
[Fig.1] [Fig. 1] is a perspective view from above of a first embodiment of container lid according to the invention, with closed through opening;
[Fig.2][Fig.2] is a longitudinal cross-section view of [Fig.1];
[Fig.3][Fig.3] is a longitudinal cross-section view of [Fig.1] with opened through opening;
[Fig.4][Fig.4] is a longitudinal cross-section view of a second embodiment of container lid according to the invention, with closed through opening; and [Fig.5][Fig.5] is a perspective view of a detail in [Fig.4].

### Description of Embodiments

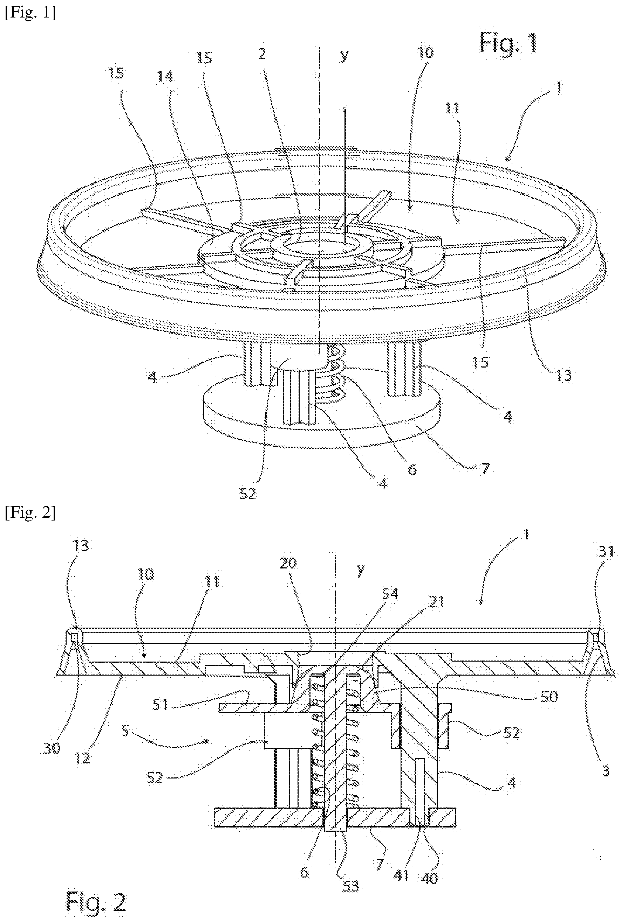

Reference is made initially to Figs. 1 and 2 which are respectively a perspective view from above and a longitudinal cross-section view of a first embodiment of container lid according to the invention, with closed through opening. For simplicity the container is not shown.

A container lid 1 is equipped with a closable through opening, indicated as 2. The through opening 2 is represented in the center of the container lid 1 with the y axis perpendicular to the lid itself. The container lid 1 is shown in a circular shape but could also be of a different shape. Likewise, the through opening 2, for symmetry and for construction practicality, is represented centrally in the container lid, but this should not be understood as a limitation to its position on the container lid itself.

The container lid 1 is made with a lid wall 10 having an upper side 11 and a lower side 12, and an edge 13. The edge 13 of the container lid 1 has a V-shaped cross-section facing downwards, as better shown in [Fig.2], so as to create a guide 3, suitable for applying pressure to the rim of a container not shown. The guide 3 ends at the bottom of the V-shaped cross-section with a protrusion 30 and a recess 31 to create a hermetic seal between the lid and the container.

Alternatively, even if not shown, the container lid 1 could have an edge suitable for threaded coupling with a container with screw closure.

The lid wall 10 preferably has a central ridge 14 and a plurality of radial ribs 15.

The through opening 2, obtained in the wall 10 of the container lid 1, protrudes inferiorly from the central ridge 14 of the lid wall 10 with a first cylindrical section 20 and a second frustoconical section 21.

A plurality of elongated elements, three in number in the figures where they are generally indicated with 4, are made in one piece with the lid wall 10 and extend from its lower side 12 along directions parallel to the y axis of the through opening 2. The y axis is essentially the central vertical axis of the container lid. The elongated elements 4 have a free end, generically indicated as 40.

The closure of the through opening 2 is obtained by means of a closing member, indicated generally as 5. The closing member 5 is of a sliding type along the axis y and facing the through opening 2. The closing member 5 has a plug 50 with a cap shape mating, in closed conditions, hermetically sealed with the second frustoconical section 21 of the through opening 2. The external profile of the plug 50 extends peripherally with a flat portion 51 in which a plurality of sliding sleeves is provided, indicated generically as 52. The sliding sleeves 52 have an internal cavity with a cross-section corresponding to that of the elongated elements 4. For example, as shown in the figures, the elongated elements 4 can have a cross-shaped section. Hence, the sliding sleeves 52 have a cross-shaped internal cavity. This ensures the constant directionality of the plug 50 with respect to the through opening 2.

Furthermore, the closing member 5 is equipped with a spring guide rod 53, extending downwards. A spiral spring 6 is inserted in the spring guide rod 53, and abuts the plug 50, preferably in a cavity 54 thereof, and a counteracting plate 7. The counteracting plate 7 is provided with a through hole 70 for the spring guide rod 53. The counteracting plate 7 is rigidly connected to the free ends 40 of the plurality of elongated elements 4. The rigid connection of each elongated element 4 with the counteracting plate 7 can be achieved by means of threaded coupling, such as a screw, not shown, inserted in a hole 41 of the elongated element 4. Alternatively, the counteracting plate 7 is adapted to be joined to the elongated elements 4 with means selected among gluing means, forcing means, welding means.

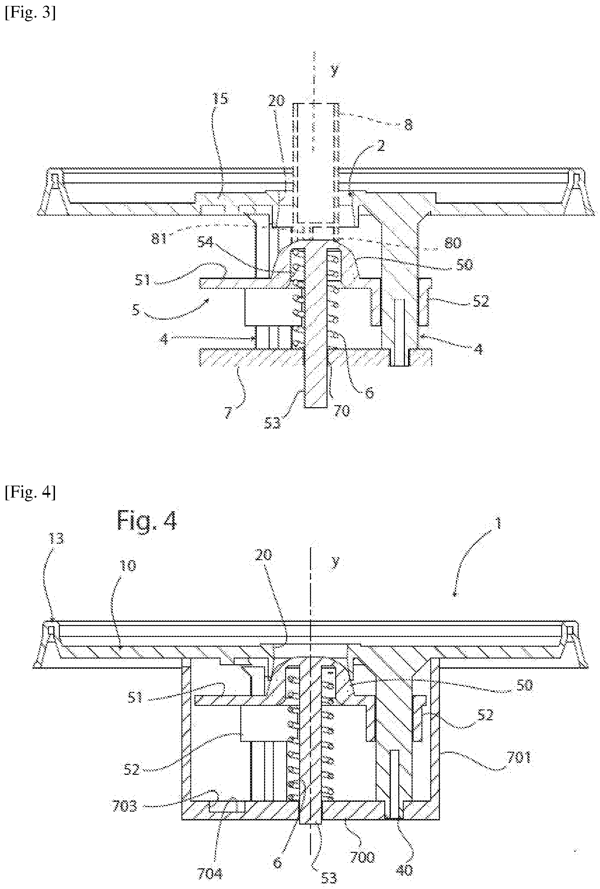

Reference is made now to [Fig.3], which is a longitudinal cross-section of [Fig.1] with opened through opening 2. Indicated with dashed lines as 8 is a nozzle of a machine for filling a container closed with the lid according to the invention. The nozzle 8 is moved by the machine downwards along the y axis of the through opening 2. Its lower end 80 causes the plug 50 to move downwards against the counteracting action of the spring 6, abutting with a first abutment on the plug 50 and, with a second abutment, on the counteracting plate 7. The lower end 80 of the nozzle 8 has a plurality of openings indicated generically as 81 to allow the lateral flow of the fixative-preservative substance inside the container which is not shown. At the end of the dispensing operation, the upward movement of the nozzle 8 brings the plug 50 back to its closed position against the frustoconical wall 21 of the through opening 2, as shown in [Fig.2].

In [Fig.4] which is a longitudinal cross-section view of a second embodiment of container lid 100 according to the invention, with closed through opening, the same reference numbers are used to represent identical parts to those of the first embodiment. The difference between the two embodiments consists in that the counteracting plate 700 extends upwards in a cylindrical wall 701, orthogonal to the counteracting plate 700.

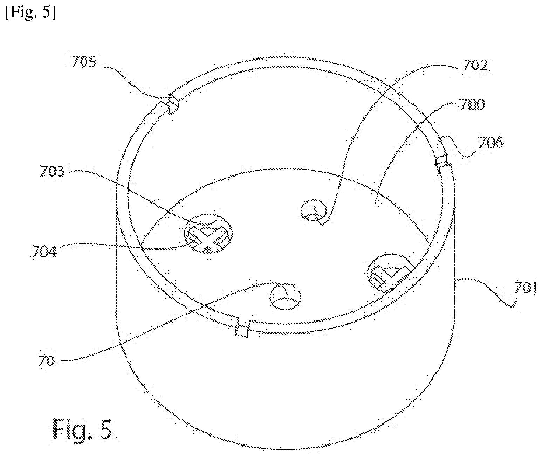

[Fig.5] is a perspective view of the counteracting plate 700 with the cylindrical wall 701. As in the first embodiment, the counteracting plate 700 is provided with a through hole 70 for the spring guide rod 53. The counteracting plate 700 is rigidly connected to the free ends of the plurality of elongated elements 4 in holes 702 into which the end 40 of each elongated element 4 is inserted. Similarly, the connecting means of the counteracting plate 700 can be threaded means, such as screws, as well as gluing means, forcing means, welding means. Indicated as 703 is a plurality of liquid passage openings suitable to allow the passage of the fixative-preservative substance. Cross elements 704 are shown inside the liquid passage openings 703 to prevent the passage of tissue samples in the space delimited by the counteracting plate 700 and the cylindrical wall 701. The upper edge 706 of the cylindrical wall 701 has recesses 705 with a vent function.

The two embodiments, as described, allow a different inflow of fixative-preservative substance. According to the first embodiment of the container lid 1 the fixative-preservative substance is distributed directly to the container. According to the second embodiment of the container lid 100, the fixative-preservative substance is directed first inside a chamber consisting of the contrast plate 700 and the cylindrical wall 701 and then the fixative-preservative substance flows towards a base of the container not shown through the holes 704 of the counteracting plate 700.

## Claims

1. A container lid (1; 100) provided with a closable through opening (2), the container lid (1; 100) having a through opening (2) with axis (y) perpendicular to the container lid (1; 100), and a closing member (5) that is spring loaded along an axis coincident with the axis (y) of the through opening (2), **characterized in that**:
- the container lid (1; 100) is configured with a lid wall (10) having an upper side (11) and a lower side (12), and an edge (13),
- the through opening (2) is made in the lid wall (10),
- a plurality of elongated elements (4) in one piece with the lid wall (10) extend from the wall lower side (12) to their free end (40), along directions parallel to said axis (y) of the through opening (2),
- the closing member (5) is equipped with
- a spring guide rod (53) extending downwards,
- a plurality of sliding sleeves (52) having an internal cross-section corresponding to that of the elongated elements (4),
- a plug (50) mating hermetically with the through opening (2) in a closed condition, and
- a coil spring (6) inserted in the spring guide rod (53), with a first abutment against the plug (50),
- a counteracting plate (7; 700) as a second abutment of the coil spring (6) is equipped with a through hole (70) for the spring guide rod (53), the counteracting plate (7; 700) being connected rigidly to the free ends (40) of the elongated elements (4).

2. The container lid (1; 100) according to claim 1, wherein the through opening (2) protrudes inferiorly from the lid wall (10) with a first cylindrical portion (20) and a second frustoconical portion (21), and the plug (50) of the closing member (5), having a cap like shape, mates hermetically with said second frustoconical portion (21) of the through opening (2).

3. The container lid (1; 100) according to claim 1, wherein the lid wall (10) has integrally a central ridge (14) and a plurality of radial ribs (15).

4. The container lid (1; 100) according to claim 1, wherein the edge (13) of the container lid (1; 100) has a guide (3) with a V-shaped cross-section facing downwards.

5. The container lid (100) according to claim 1, wherein the counteracting plate (700) extends orthogonally upwards in a cylindrical wall (701).

6. The container lid (100) according to claim 5, wherein the counteracting plate (700) is further provided with liquid passage openings (703).

7. The container lid (100) according to claim 5, wherein the cylindrical wall (701) is provided at the top with vent recesses (705).

8. The container lid (1; 100) according to claim 1, wherein the counteracting plate (7; 700) is adapted to be joined with screws to the free ends (40) of the plurality of elongated elements (4).

9. The container lid (1; 100) according to claim 1, wherein the counteracting plate (7; 700) is adapted to be joined to the elongated elements (4) with means selected from a group comprising gluing means, forcing means, welding means.

## Patentansprüche

1. Behälterdeckel (1; 100) mit verschließbarer Durchgangsöffnung (2), wobei der Behälterdeckel (1; 100) eine Durchgangsöffnung (2) mit einer Achse (y) aufweist, die senkrecht zum Behälterdeckel (1; 100) angeordnet ist, und ein Verschlusselement (5), das entlang einer Achse, die mit der Achse (y) der Durchgangsöffnung (2) übereinstimmt, gefedert ist, **dadurch gekennzeichnet, dass**
- der Behälterdeckel (1; 100) mit einer Deckelwand (10) ausgelegt ist, die eine obere Seite (11) und eine untere Seite (12) und einen Rand (13) aufweist;
- die Durchgangsöffnung (2) in der Deckelwand (10) ausgebildet ist;
- eine Vielzahl von länglichen Elementen (4) sich in einem Stück mit der Deckelwand (10) von der unteren Wandseite (12) zu ihrem freien Ende (40) entlang Richtungen erstrecken, die parallel zur Achse (y) der Durchgangsöffnung (2) angeordnet sind;
- das Verschlusselement (5) ausgestattet ist mit
- einem Federführungsstab (53), der sich nach unten erstreckt;
- einer Vielzahl von Schiebehülsen (52), die einen Innenquerschnitt aufweisen, der dem der länglichen Elemente (4) entspricht;
- einem Stopfen (50), der hermetisch passend zur Durchgangsöffnung (2) in einem geschlossenen Zustand ausgeführt ist, und
- einer Spulenfeder (6), die in den Federführungsstab (53) mit einem ersten Anschlag gegen den Stopfen (50) eingefügt ist;
- eine gegenwirkende Platte (7; 700) als zweitem Anschlag der Spulenfeder (6) mit einem Durchführungsloch (70) für den Federführungsstab (53) ausgestattet ist, wobei die gegenwirkende Platte (7; 700) steif mit den freien Enden (40) der länglichen Elemente (4) verbunden ist.

2. Behälterdeckel (1; 100) nach Anspruch 1, wobei die Durchführungsöffnung (2) unterseitig aus der Deckelwand (10) mit einem ersten zylindrischen Abschnitt (20) und einem zweiten kegelstumpfförmigen Abschnitt (21) hervorsteht und der Stopfen (50) des Verschlusselements (5), aufweisend eine kapselähnliche Form, hermetisch passend zum zweiten kegelstumpfförmigen Abschnitt (21) der Durchführungsöffnung (2) ausgeführt ist.

3. Behälterdeckel (1; 100) nach Anspruch 1, wobei die Deckelwand (10) integral eine mittige Erhöhung (14) und eine Vielzahl von radialen Rippen (15) aufweist.

4. Behälterdeckel (1; 100) nach Anspruch 1, wobei der Rand (13) des Behälterdeckels (1; 100) eine Führung (3) mit einem V-förmigen Querschnitt aufweist, der nach unten gewandt ist.

5. Behälterdeckel (100) nach Anspruch 1, wobei sich die gegenwirkende Platte (700) rechtwinkelig in einer zylindrischen Wand (701) nach oben erstreckt.

6. Behälterdeckel (100) nach Anspruch 5, wobei die gegenwirkende Platte (700) zudem mit Flüssigkeitsdurchströmungsöffnungen (703) versehen ist.

7. Behälterdeckel (100) nach Anspruch 5, wobei die zylindrische Wand (701) an der Oberseite mit Belüftungsausnehmungen (705) versehen ist.

8. Behälterdeckel (1; 100) nach Anspruch 1, wobei die gegenwirkende Platte (7; 700) dazu geeignet ist, mit Schrauben mit den freien Enden (40) der Vielzahl von länglichen Elementen (4) zusammengefügt zu werden.

9. Behälterdeckel (1; 100) nach Anspruch 1, wobei die gegenwirkende Platte (7; 700) dazu geeignet ist, mit den länglichen Elementen (4) mit Mitteln zusammengefügt zu werden, die aus einer Gruppe, umfassend Klebemittel, Forciermittel, Schweißmittel, ausgewählt sind.

## Revendications

1. Couvercle (1 ; 100) de récipient pourvu d'une ouverture traversante (2) pouvant être fermée, le couvercle (1 ; 100) de récipient comportant une ouverture traversante (2) dont l'axe (y) est perpendiculaire au couvercle (1 ; 100) de récipient, et un élément de fermeture (5) étant chargé par un ressort le long d'un axe coïncidant avec l'axe (y) de l'ouverture traversante (2), **caractérisé en ce que** :
- le couvercle (1 ; 100) de récipient est configuré avec une paroi (10) de couvercle comportant un côté supérieur (11) et un côté inférieur (12), et un bord (13),
- l'ouverture traversante (2) est réalisée dans la paroi (10) de couvercle,
- une pluralité d'éléments allongés (4), d'un seul tenant avec la paroi (10) de couvercle, s'étendent du côté inférieur (12) de la paroi jusqu'à leur extrémité libre (40), le long de directions parallèles audit axe (y) de l'ouverture traversante (2),
- l'élément de fermeture (5) est équipé
- d'une tige de guidage (53) à ressort s'étendant vers le bas,
- d'une pluralité de manchons coulissants (52) comportant une section transversale interne correspondant à celle des éléments allongés (4),
- d'un bouchon (50) s'accouplant hermétiquement à l'ouverture traversante (2) dans une condition fermée, et
- un ressort hélicoïdal (6) inséré dans la tige de guidage (53) à ressort, avec une première butée contre le bouchon (50),
- une plaque antagoniste (7 ; 700), comme étant une deuxième butée du ressort hélicoïdal (6), est équipée d'un trou traversant (70) pour la tige de guidage (53) à ressort, la plaque antagoniste (7 ; 700) étant reliée de manière rigide aux extrémités libres (40) des éléments allongés (4).

2. Couvercle (1 ; 100) de récipient selon la revendication 1, dans lequel l'ouverture traversante (2) dépasse inférieurement de la paroi (10) de couvercle avec une première partie cylindrique (20) et une deuxième partie tronconique (21), et le bouchon (50) de l'élément de fermeture (5), ayant une forme de capuchon, s'accouple hermétiquement avec ladite deuxième partie tronconique (21) de l'ouverture traversante (2).

3. Couvercle (1 ; 100) de récipient selon la revendication 1, dans lequel la paroi (10) de couvercle comporte intégralement une arête centrale (14) et une pluralité de nervures radiales (15).

4. Couvercle (1 ; 100) de récipient selon la revendication 1, dans lequel le bord (13) du couvercle (1 ; 100) de récipient comporte un guide (3) à section transversale en « V » orientée vers le bas.

5. Couvercle (100) de récipient selon la revendication 1, dans lequel la plaque antagoniste (700) s'étend orthogonalement vers le haut dans une paroi cylindrique (701).

6. Couvercle de récipient (100) selon la revendication 5, dans lequel la plaque antagoniste (700) est de plus pourvue d'ouvertures de passage (703) de liquide.

7. Couvercle (100) de récipient selon la revendication 5, dans lequel la paroi cylindrique (701) est pourvue au sommet d'évidements d'évent (705).

8. Couvercle (1 ; 100) de récipient selon la revendication 1, dans lequel la plaque antagoniste (7 ; 700) est adaptée pour être jointe par des vis aux extrémités libres (40) de la pluralité d'éléments allongés (4).

9. Couvercle (1 ; 100) de récipient selon la revendication 1, dans lequel la plaque antagoniste (7 ; 700) est adaptée pour être jointe aux éléments allongés (4) à l'aide de moyens choisis dans un groupe comprenant des moyens de collage, des moyens de forçage, des moyens de soudage.
